# FASCICULE DE BREVET EUROPEEN

(11) **EP 0 884 948 B1**
(45) Date de publication et mention de la délivrance du brevet: **19.12.2001**
(21) Numéro de dépôt: 97907152.9
(22) Date de dépôt: 28.02.1997
(51) Int. Cl.: A01N 47/44

(54) **COMPOSITIONS ANTISEPTIQUES A BASE DE CHLOROBUTANOL ET DE CHLORHEXIDINE**
ANTISEPTISCHE ZUSAMMENSETZUNGEN AUF BASIS VON CHLOROBUTANOL UND CHLORHEXIDIN
ANTISEPTIC COMPOSITIONS CONTAINING CHLOROBUTANOL AND CHLORHEXIDINE

(30) Priorité: 04.03.1996 FR 9602685
(43) Date de publication de la demande: 23.12.1998
(73) Titulaire: PIERRE FABRE MEDICAMENT, 92100 Boulogne-Billancourt (FR)
(72) Inventeur: MROZ, Christian, F-31520 Ramonville-Saint-Agne (FR); SEGONDS, Rolland, F-31000 Toulouse (FR)
(74) Mandataire: Ahner, Francis
(86) Numéro de dépôt international: FR9700362
(87) Numéro de publication internationale: WO9732479

(56) Documents cités:
- "Dictionnaire Vidal" 1995 , EDITIONS DU VIDAL , PARIS, FR XP002018291 71e édition * page 521: ELUDRIL solution *

## Description

La présente invention concerne des compositions antiseptiques associant la Chlorhexidine ou l'un de ses sels et le Chlorobutanol dans des proportions particulières conduisant à une synergie sur le plan de l'activité antiseptique. Il peut s'agir en particulier de préparations pharmaceutiques topiques, mais également de produits de désinfection notamment du matériel médical.

La Chlorhexidine est un antiseptique local largement utilisé en application topique (plaies, soins ORL, irrigations des cavités internes, etc) ou comme désinfectant. Elle peut être associée à d'autres substances antimicrobiennes comme de l'alcool ou des ammoniums quaternaires. Les concentrations minimales en sels de Chlorhexidine avoisinnent alors 100 mg, voire 50 mg pour 100 ml.

Le Chlorobutanol est surtout employé comme analgésique. Ses propriétés antimicrobiennes de type bactériostatique permettent aussi son utilisation comme agent de conservation des compositions pharmaceutiques.

Le Chlorobutanol est utilisé dans les compositions pharmaceutiques à des concentrations qui sont très variables (de 25 à 500 mg pour 100 ml). Il peut être associé à de véritables antiseptiques, mais seul ELUDRIL SOLUTION® (Pierre Fabre Médicament) l'associe à de la Chlorhexidine. La formule commercialisée en France contient 100 mg pour 100 ml de Digluconate de Chlorhexidine et 100 mg pour 100 ml de Chlorobutanol hémihydraté. Cette solution s'utilise diluée au demi ou au quart et l'activité antiseptique est attribuée à la Chlorhexidine. Seule une activité sédative est attribuée au Chlorobutanol. Une variante commercialisée au Royaume Uni contient la même quantité de Chlorhexidine, mais 500 mg pour 100 ml de Chlorobutanol hémihydraté. Les propriétés observées pour les deux composants sont les mêmes et il s'agit toujours d'une solution à diluer.

Or, de façon surprenante, il a été trouvé que l'association de Chlorhexidine ou de l'un des sels avec le Chlorobutanol, sous forme anhydre ou hémihydratée, lorsqu'elle était convenablement réalisée, permettait une potentialisation de l'activité antimicrobienne de ces deux principes. Cette potentialisation s'exerce aussi bien sur les bactéries gram - négatives que sur les bactéries gram positives. Elle permet dans certains cas de réduire mille fois la dose nécessaire en Chlorhexidine pour obtenir un résultat équivalent.

Conformément à la présente invention, la composition antiseptique à base d'une association de Chlorobutanol anhydre ou hémihydraté avec de la Chlorhexidine ou l'un de ses sels est caractérisée en ce que le Chlorobutanol est présent à une concentration minimale d'environ 200 mg pour 100 ml, la Chlorhexidine ou son sel est présent à une concentration, exprimée en Chlorhexidine base, de 0,28 mg à 14 mg pour 100 ml, et le rapport pondéral entre le Chlorobutanol et la Chlorhexidine exprimée en base libre est compris entre 14 et 1800.

Selon une caractéristique avantageuse de la présente invention, le Chlorobutanol est présent à une concentration d'environ 500 mg pour 100 ml.

On a tout d'abord déterminé l'activité antimicrobienne du Chlorobutanol en solution aqueuse à 500 mg pour 100 ml. Pour cela, deux méthodologies ont été utilisées :
- La méthodologie proposée par la Pharmacopée Européenne IIème édition dans l'annexe VIII.14 et intitulée "Efficacité de la conservation antimicrobienne". Dans cet essai, des suspensions de germes sont maintenues en contact avec le produit à évaluer jusqu'à 28 jours à 20-25°C. Le nombre de cellules survivant à ce contact est suivi en fonction de la durée de celui-ci. L'efficacité du pouvoir de conservation est exprimée en logarithme de réduction (logR) des populations de germes-test. Les souches à essayer sont stipulées par la Pharmacopée Européenne.
Ce type d'essai réalisé sur le Chlorobutanol en solution aqueuse de 500 mg pour 100 ml a conduit aux résultats reportés dans le tableau 1 ci-après.

**TABLEAU 1**

| Réduction logarithmique (LOG R) des populations microbiennes en fonction du temps de contact avec une solution aqueuse à 500 mg pour 100 ml de Chlorobutanol | | | | |
|---|---|---|---|---|
| Souches test | Temps de contact | | | |
| | 48h | 7j | 14j | 28j |
| Pseudomonas acruginosa ATCC 9027 | >4,4 | >4,4 | >4,4 | >4,4 |
| Escherichia coli ATCC 8739 | >4,3 | >4,3 | >4,3 | >4,3 |
| Staphylococcus aureus ATCC 6538 | >4,1 | >4,1 | >4,1 | >4,1 |
| Candida albicans ATCC 10231 | >4 | >4 | >4 | >4 |
| Aspergillus niger ATCC 16404 | 0,4 | 1,8 | 2,4 | 2,6 |

- La méthodologie décrite par la Pharmacopée Française Xème édition dans la monographie "Antiseptica" de Janvier 1990 et qui permet de déterminer l'activité antiseptique de préparations pharmaceutiques. Elle précise qu'un antiseptique doit avoir une activité telle que la concentration en microorganismes vivants d'une suspension définie de microorganismes par millilitre soit diminuée de 10⁵ fois par contact avec celui-ci pendant une durée variant selon l'utilisation et les indications du fabricant, à une température de 32°C. La méthodologie à suivre pour cette détermination d'activité est décrite dans cette monographie ainsi qu'une liste non limitative de germes à essayer.

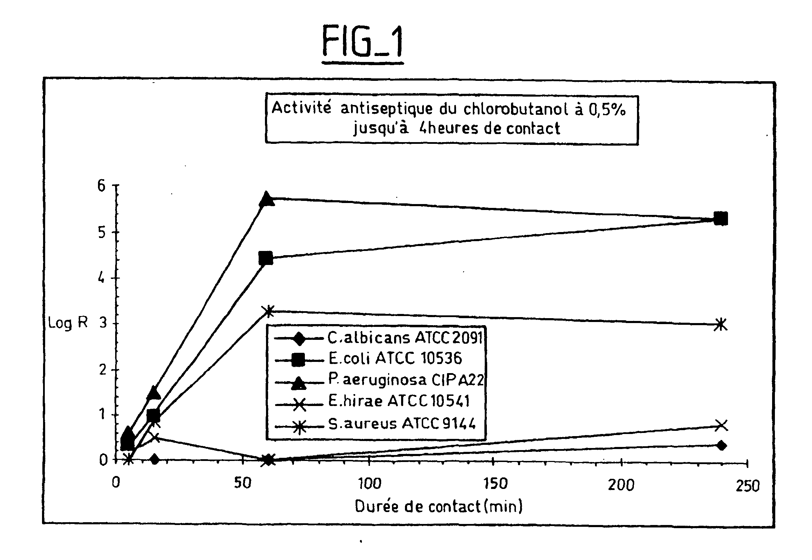

Le contact a été préalablement défini à 5 minutes sur 9 souches microbiennes (voir tableau 2 ci-après), puis a été prolongé jusqu'à 4 heures pour 5 d'entre elles (voir figure 1 annexée).

**TABLEAU 2**

| Activité antiseptique du Chlorobutanol à 500 mg pour 100 ml après 5 min de contact à 32° C (exprimée en log R) | |
|---|---|
| P. aeruginosa A22 | 0,6 |
| E coli ATCC 10536 | 0,3 |
| S. aureus ATCC 9144 | 0 |
| E. hirae ATCC 10541 | 0 |
| S. mutans ATCC 25175 | 0 |
| S. sobrinus ATCC 33478 | 0,5 |
| A. actinomycetemcomitans ATCC 33384 | 1,3 |
| P. intermedia NCTC 93336 | 1,1 |
| C. albicans ATCC 2091 | 0 |

On observe que le niveau d'exigence demandé par la Pharmacopée Française n'est atteint que sur quelques souches du spectre microbien essayé.

Les bactéries gram-négatives semblent être les germes les plus sensibles. Toutefois, la durée de contact nécessaire pour obtenir les 5 logarithmes de réduction est d'environ une heure.

Pour les autres souches, un contact de 4 heures est toujours insuffisant. Les résultats précédents (tableau 1) obtenus sur des souches équivalentes laissent à penser que la durée de contact nécessaire serait d'au moins 2 jours.

En conclusion, le Chlorobutanol possède une activité antimicrobienne qui est compatible avec son utilisation à la concentration de 500 mg pour 100 ml comme agent de conservation dans les préparations pharmaceutiques. Toutefois, ces propriétés antimicrobiennes jusqu'à cette concentration et même sur des durées d'application très longues (au moins 4 heures) sont nettement insuffisantes pour revendiquer une activité antiseptique selon les termes de la Pharmacopée Française .

L'activité antimicrobienne de la Chlorhexidine a été déterminée de la même façon.

On sait que l'efficacité des agents antiseptiques est liée à de nombreux paramètres : concentration initiale en actif, mode et durée d'application des produits, température de contact, nature des agents microbiens à éliminer et état physiologique de ceux-ci, présence d'agents neutralisants de l'antiseptique considéré au niveau de la zone d'application. Les concentrations en sels de Chlorhexidine dans les préparations antiseptiques peuvent ainsi varier de 50 à 4000 mg pour 100 ml selon l'usage et la présentation de ces produits (solutions pour application locale utilisées pour la préparation des champs opératoires ou l'antiseptie de peaux lésées, gels, bains de bouche, collutoires pour le traitement des affections bucco-dentaires, etc).

L'activité de la Chlorhexidine (sous la forme de ses sels) a été quantifiée en fixant les plus importants de ces paramètres. En ce qui concerne la température de contact (32°C) et le spectre microbien à essayer, les exigences de la Pharmacopée Française ont été reprises. La durée de contact a été fixée à une valeur volontairement courte (5 min) car très discriminante ; elle est aussi en relation avec l'usage thérapeuthique recherché. Les essais ont été réalisés en l'absence d'agents interférant avec l'action de la Chlorhexidine. Dans ces conditions précises de contact, on a établi les concentrations minimales nécessaires pour réduire de 10⁵ fois la concentration en microorganismes vivants selon la définition de la Pharmacopée Française (tableau 3). Les résultats sont exprimés en Chlorhexidine base, ils sont équivalents pour chacun des sels testés (digluconate et diacétate).

**TABLEAU 3**

| Concentrations minimales antiseptiques de la Chlorhexidine base (mg pour 100 ml) pour un contact de 5 min à 32° C | |
|---|---|
| P. aeruginosa A22 | 2,8 |
| E coli ATCC 10536 | 1,4 |
| S. aureus ATCC 9144 | 28 |
| E. hirae ATCC 10541 | 280 |

La Chlorhexidine agit donc de manière très rapide et à faible concentration sur un large spectre bactérien. Les valeurs varient d'un germe à l'autre .

Pour mettre en évidence la synergie de l'activité antiseptique entre la Chlorhexidine et le Chlorobutanol, les essais réalisés précédemment ont été répétés en modifiant un seul des paramètres. Il s'agit cette fois de déterminer les concentrations minimales nécessaires en Chlorhexidine en solution, non plus dans l'eau, mais dans une solution aqueuse à 500 mg pour 100 ml de Chlorobutanol pour réduire de 10⁵ fois la concentration en microorganismes vivants .

Les résultats ainsi obtenus ont été comparés aux résultats précédemment obtenus avec la Chlorhexidine seule (tableau 3). Le rapport entre les 2 scores a été calculé pour chaque souche, déterminant ainsi pour chaque souche microbienne un facteur de potentialisation (tableau 4).

**TABLEAU 4**

| Concentrations minimales antiseptiques de la chlorhixidine base (mg/100 ml) pour un contact de 5 min à 32° C | | | |
|---|---|---|---|
| Souches testées | dans l'eau (rappel tableau 3) | dans le Chlorobutanol (500 mg pour 100 ml) | Facteur de potentialisation |
| P. aeruginosa A22 | 2,8 | 0,056 | 50 fois |
| E. coli ATCC 10536 | 1,4 | 0,14 | 10 fois |
| S. aureus ATCC 9144 | 28 | 0,28 | 100 fois |
| E. hirae ATCC 10541 | 280 | 0,28 | 1000 fois |

La potentialisation de l'activité antiseptique de la Chlorhexidine par le Chlorobutanol s'exerce sur toutes les souches bactériennes testées. Elle permet de réduire considérablement (jusqu'à 1000 fois dans certains cas) les concentrations nécessaires en Chlorhexidine. Ceci est tout particulièrement vrai sur les souches gram-positives (S.aureus ATCC 9144 et E. hirae ATCC 10541) qui étaient initialement les moins sensibles à la Chlorhexidine.

Des essais ont été réalisés en présence de concentrations variables de Chlorhexidine et de Chlorobutanol. Ce type d'essai a permis d'observer (tableau 5) que la quantité de Chlorobutanol permettant la potentialisation de l'activité de la Chlorhexidine est corrélée à la quantité de Chlorhexidine déjà présente dans la préparation. Ainsi donc:
- la présence de Chlorobutanol n'est nécessaire que si la concentration de Chlorhexidine dans la préparation est insuffisante pour exprimer une activité de type antiseptique ; cette concentration limite efficace en Chlorhexidine est variable selon la souche microbienne testée comme démontré précédemment,
- la dose à appliquer en Chlorobutanol est inversement proportionnelle à la dose de Chlorhexidine présente dans la préparation et croit rapidement vers des valeurs supérieures ou égales à 250 mg pour 100 ml ; le rapport pondéral entre la Chlorobutanol et la Chlorhexidine se situe dans un intervalle large, à partir de valeurs proches de 10 jusqu'à des valeurs supérieures à 1 000 dans certains cas.

**TABLEAU 5**

| Concentration initiale en Chlorhexidine base (mg pour 100 ml) | Concentration minimale en Chlorobutanol (mg pour 100 ml) qu'il faut associer pour obtenir une réduction des populations > 10⁵ fois après 5 min de contact à 32° C et selon la souche essayée | |
|---|---|---|
| | E hirae ATCC 10541 | P. aeruginosa A 22 |
| 280 | 0 | 0 |
| 5,6 | 50 | 0 |
| 2,8 | 250 | 0 |
| 0,28 | 500 | 250 |

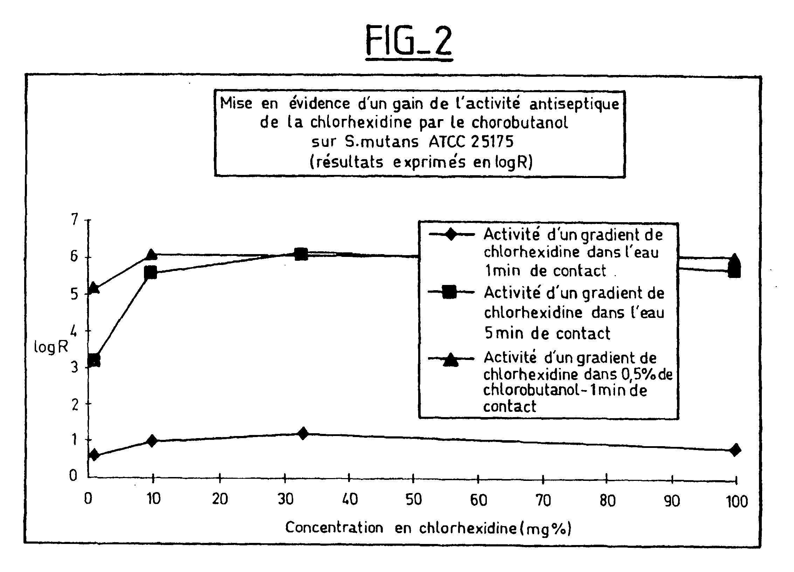

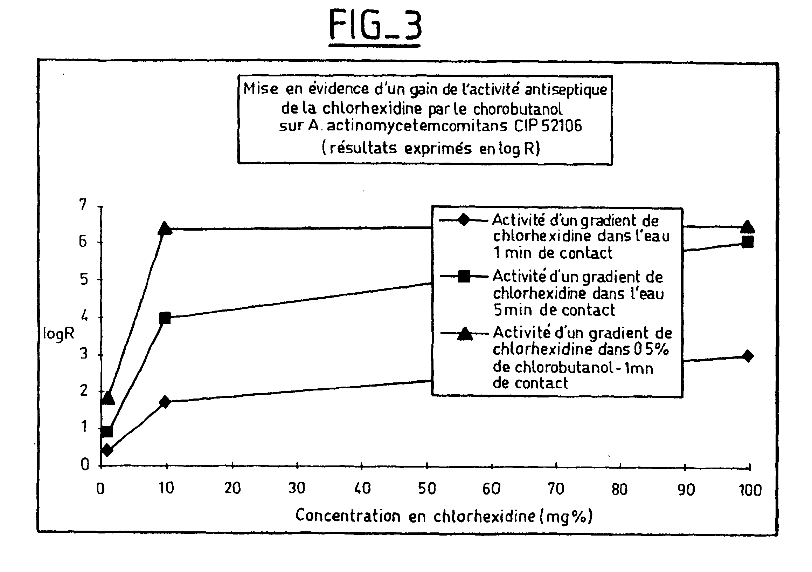

Des essais sur différentes durées de contact montrent que la potentialisation de la Chlorhexidine par le Chlorobutanol permet de réduire le temps nécessaire à l'expression de l'activité de la Chlorhexidine. Ceci est particulièrement appréciable, par exemple, dans le cadre des bains de bouche où la durée d'application est très courte. Ceci a été démontré sur deux souches de la flore buccale : Streptococcus mutans ATCC 25175, souche cariogène (voir figure 2 annexée) et Actinobacillus actinomycetemcomitans CIP 52106 (voir figure 3 annexée), souche responsable de maladies parodontales.

Ainsi, l'association Chlorhexidine - Chlorobutanol permet d'obtenir un pouvoir antiseptique en 1 min équivalent (cas de S. mutans), voire supérieur (cas d'A. actinomycetemcomitans) à celui obtenu après 5 min.de contact avec la Chlorhexidine seule. Ceci est particulièrement vrai dans le cas où les doses appliquées en Chlorhexidine sont très faibles (inférieures à 5,6 mg pour 100 ml). Dans les mêmes conditions de contact le Chlorobutanol seul n'exerce pas ou peu d'activité. En effet, en présence de 500 mg pour 100 ml de Chlorobutanol, le logarithme de réduction des populations de S. mutans est nul après 5 min de contact à 32° C (figure 2 annexée) et de 1,3 pour A. actinomycetemcomitans (figure 3 annexée).

On se trouve donc bien en présence d'une potentialisation et non pas d'une additivité d'activité .

En conclusion, après avoir mis en évidence l'absence de propriétés antiseptiques de la part du Chlorobutanol et les limites de l'activité antiseptique de la Chlorhexidine, la présente invention démontre que l'ajout de Chlorobutanol dans des préparations à base de Chlorhexidine ou de ses sels permet d'augmenter de manière importante l'activité de cet antiseptique. La' potentialisation se traduit par une diminution de la concentration utile en Chlorhexidine et / ou une diminution du temps de contact utile à son action. Cette potentialisation s'exprime aussi par le fait que, pour une concentration donnée, le spectre d'activité de la Chlorhexidine est élargi grace à l'augmentation de la sensibilité des germes à cet antiseptique .

Cette potentialisation permet la formulation de préparations antiseptiques très faiblement dosées en Chlorhexidine (par exemple : entre 0,5 et 25 mg pour 100 ml de Digluconate de Chlorhexidine) et contenant du Chlorobutanol (par exemple: de 200 à 500 mg pour 100 ml de Chlorobutanol hémi-hydraté) . Ces préparations sont efficaces même avec des durées de contact très courtes (de l'ordre de 1 min). Le rapport pondéral optimum Chlorobutanol/Chlorhexidine base est compris entre 14 et 1800. Cette base active est transposable à toutes les formes galéniques : liquides, pâteuses, ou solides.

La présente invention offre de nombreux avantages :
- Elle permet, par l'ajout de Chlorobutanol, de renforcer l'activité antimicrobienne de compositions pharmaceutiques à base de Chlorhexidine (ou de ses sels hydrochloriques, acétiques, gluconiques, etc). Ceci est particulièrement appréciable pour les produits dont les temps d'application sont obligatoirement courts (bains de bouches, produits à rincer, etc), pour les compositions contenants des substances peu compatibles avec la Chlorhexidine et ses sels (tensio-actifs anioniques, surfactifs non ioniques, etc) ou lorsque l'activité des compositions doit s'exercer en présence d'agents interférants (protéines, phospholipides, etc).
- Elle permet, en diminuant la concentration en Chlorhexidine ou ses sels, de réduire leurs effets secondaires (coloration de la langue et des dents, irritations cutanées, eczémas allergiques, photosensibilisation, etc).
- Elle permet l'élargissement du spectre d'activité des compositions pharmaceutiques associant ces deux agents antimicrobiens.

Bien entendu, la présente invention est applicable à un grand nombre de formes pharmaceutiques antiseptiques telles que :
- solutions
- solutions moussantes
- émulsions ou pommades
- gels
- savons
- sticks
- bains de bouches et collutoires
- pâtes dentifrices
- comprimés
- comprimés hydro-dispersibles
- tablettes / comprimés à sucer ou à croquer
- sprays
- shampooing

A titre d'illustration, on mentionnera quelques exemples de formes pharmaceutiques :

| Exemple de solution antiseptique | pour 100 ml : |
|---|---|
| Digluconate de Chlorhexidine | de 0,5 à 25 mg |
| Chlorobutanol hémihydraté | de 200 à 500 mg |
| Parfum | de 0,05 à 3 ml |
| Eau purifiée | qsp 100 ml |

| Exemple de bain de bouche | pour 100 ml : |
|---|---|
| Digluconate de Chlorhexidine | de 0,5 à 25 mg |
| Chlorobutanol hémihydraté | de 200 à 500 mg |
| Alcool éthylique à 95% | de 1 à 30 ml |
| Aromes exemples : | |
| - Menthol | de 50 mg à 150 mg |
| - Solution alcoolique d'essence de Menthe | de 0,5 à 2 ml |
| Eau purifiée | qsp 100 ml |

| Exemple de crème antibactérienne | pour 100 ml : |
|---|---|
| Diacétate de Chlorhexidine | de 0,35 à 17,5 mg |
| Chlorobutanol hémihydraté | de 200 à 500 mg |
| Huile de Vaseline épaisse | de 1 à 20 g |
| Cire émulsionnante à base d'alcool gras | de 1 à 20 g |
| Antioxydant | de 10 à 500 mg |
| Eau purifiée | qsp 100 ml |

## Revendications

1. Composition antiseptique, en particulier préparation pharmaceutique topique à base d'une association de Chlorobutanol sous forme anhydre ou hémihydratée avec de la Chlorhexidine ou l'un de ses sels, **caractérisée en ce que** :
- le Chlorobutanol est présent à une concentration minimale d'environ 200 mg pour 100 ml,
- la Chlorhexidine ou son sel est présent à une concentration, exprimée en Chlorhexidine base, de 0,28 mg à 14 mg pour 100 ml, et
- le rapport pondéral entre le Chlorobutanol et la Chlorhexidine exprimée en base libre est compris entre 14 et 1 800.

2. Composition selon la revendication 1, **caractérisée en ce que** le Chlorobutanol est présent à une concentration d'environ 500 mg pour 100 ml.

3. Composition selon l'une des revendications 1 et 2, **caractérisée en ce que** le sel de Chlorhexidine est choisi parmi les sels acétiques, hydrochloriques et gluconiques.

4. Composition selon l'une des revendications 1 à 3, **caractérisée en ce que** le Chlorobutanol est utilisé sous la forme de son hémi-hydrate.

5. Composition selon l'une des revendications 1 à 4, **caractérisée en ce que** la Chlorhexidine ou son sel est présent à une concentration, exprimée en base libre, de 0,28 mg à 1,4 mg pour 100 ml, conférant à ladite composition un effet bactéricide.

6. Composition selon l'une des revendications 1 à 4, **caractérisée en ce que** la Chlorhexidine ou son sel est présent à une concentration, exprimée en base libre, de 1,4 mg à 14 mg pour 100 ml, conférant à ladite composition des effets bactéricide et fongicide.

7. Composition selon l'une des revendications 1 à 6, **caractérisée en ce qu'**elle comprend pour 100 ml :
- Digluconate de Chlorhexidine : de 0,5 à 25 mg,
- Chlorobutanol : de 200 à 500 mg.

8. Composition selon l'une des revendications 1 à 6, **caractérisée en ce qu'**elle comprend pour 100 ml :
- Diacétate de Chlorhexidine : de 0,35 à 17,5 mg,
- Chlorobutanol : de 200 à 500 mg.

## Patentansprüche

1. Antiseptische Zusammensetzung, insbesondere topisches pharmazeutisches Präparat, auf der Basis einer Verbindung von Chlorbutanol in wasserfreier oder hemihydratisierter Form mit Chlorhexidin oder einem seiner Salze, **dadurch gekennzeichnet, dass**:
- das Chlorbutanol in einer minimalen Konzentration von etwa 200 mg pro 100 ml vorliegt,
- das Chlorhexidin oder sein Salz in einer Konzentration, ausgedrückt als Chlorhexidin-Base, von 0,28 mg bis 14 mg pro 100 ml vorliegt und
- das Gewichtsverhältnis von Chlorbutanol zu Chlorhexidin, ausgedrückt als freie Base, zwischen 14 und 1800 einschließlich beträgt.

2. Zusammensetzung nach Anspruch 1, **dadurch gekennzeichnet, dass** das Chlorbutanol bei einer Konzentration von etwa 500 mg pro 100 ml vorliegt.

3. Zusammensetzung nach einem der Ansprüche 1 und 2, **dadurch gekennzeichnet, dass** das Salz des Chlorhexidins ausgewählt ist aus essigsauren, chlorwasserstoffsauren und gluconsauren Salzen.

4. Zusammensetzung nach einem der Ansprüche 1 bis 3, **dadurch gekennzeichnet, dass** das Chlorbutanol in Form seines Hemihydrats verwendet wird.

5. Zusammensetzung nach einem der Ansprüche 1 bis 4, **dadurch gekennzeichnet, dass** das Chlorhexidin oder sein Salz in einer Konzentration, ausgedrückt als freie Base, von 0,28 mg bis 1,4 mg pro 100 ml vorliegt, was der Zusammensetzung eine bakterizide Wirkung verleiht.

6. Zusammensetzung nach einem der Ansprüche 1 bis 4, **dadurch gekennzeichnet, dass** das Chlorhexidin oder sein Salz in einer Konzentration, ausgedrückt als freie Base, von 1,4 mg bis 14 mg pro 100 ml vorliegt, was der Zusammensetzung bakterizide und fungizide Wirkungen verleiht.

7. Zusammensetzung nach einem der Ansprüche 1 bis 6, **dadurch gekennzeichnet, dass** sie pro 100 ml umfasst:
- Chlorhexidindigluconat: 0,5 bis 25 mg,
- Chlorbutanol: 200 bis 500 mg.

8. Zusammensetzung nach einem der Ansprüche 1 bis 6, **dadurch gekennzeichnet, dass** sie pro 100 ml umfasst:
- Chlorhexidindiacetat: 0,35 bis 17,5 mg,
- Chlorbutanol: 200 bis 500 mg.

## Claims

1. Antiseptic composition, in particular a topical pharmaceutical preparation based on a combination of chlorobutanol in anhydrous or hemihydrate form with chlorhexidine or one of its salts, **characterized in that**:
- the chlorobutanol is present at a minimum concentration of about 200 mg per 100 ml,
- the chlorhexidine or its salt is present at a concentration, expressed as chlorhexidine base, of 0.28 mg to 14 mg per 100 ml, and
- the weight ratio between the chlorobutanol and chlorhexidine, expressed as a free base, is between 14 and 1 800.

2. Composition according to Claim 1, **characterized in that** the chlorobutanol is present at a concentration of about 500 mg per 100 ml.

3. Composition according to either of Claims 1 and 2, **characterized in that** the chlorhexidine salt is chosen from its acetic, hydrochloric and gluconic salts.

4. Composition according to one of Claims 1 to 3, **characterized in that** the chlorobutanol is used in the form of its hemihydrate.

5. Composition according to one of Claims 1 to 4, **characterized in that** the chlorhexidine or its salt is present at a concentration, expressed as a free base, of 0.28 mg to 1.4 mg per 100 ml, conferring a bactericidal effect on the said composition.

6. Composition according to one of Claims 1 to 4, **characterized in that** the chlorhexidine or its salt is present at a concentration, expressed as a free base, of 1.4 mg to 14 mg per 100 ml, conferring bactericidal and fungicidal effects on the said composition.

7. Composition according to one of Claims 1 to 6, **characterized in that** it comprises per 100 ml:
- chlorhexidine digluconate: from 0.5 to 25 mg,
- chlorobutanol: from 200 to 500 mg.

8. Composition according to one of Claims 1 to 6, **characterized in that** it comprises per 100 ml:
- chlorhexidine diacetate: from 0.35 to 17.5 mg,
- chlorobutanol: from 200 to 500 mg.
